# EUROPEAN PATENT APPLICATION

(11) **EP 0 801 069 A1**
(43) Date of publication of application: **15.10.1997**
(21) Application number: 97201652.1
(22) Date of filing: 07.06.1993
(51) Int. Cl.: C07D 503/00

(54) **Process for the preparation of clavulanic acid**

(30) Priority: 11.06.1992 GB 9212379; 31.10.1992 GB 9222841; 14.12.1992 GB 9226061; 17.12.1992 GB 9226282
(62) Divisional of application: 95201084.1
(71) Applicant: SMITHKLINE BEECHAM PLC, Brentford, Middlesex TW8 9EP (GB)
(72) Inventor: Cook, Michael Allen, Worthing, West Sussex BN14 8QH (GB); Wilkins, Robert Bennett, Irvine, Ayrshire, KA11 5AP (GB)
(74) Representative: Lawton, Peter Philip

(57) **Abstract**

A Process for preparing Clavulanic Acid using t-octylamine

## Description

This invention relates to a novel process for the preparation of clavulanic acid (I): 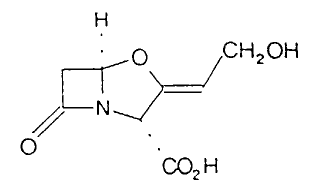 and pharmaceutically acceptable salts and esters thereof.

Clavulanic acid is normally prepared by the fermentation of a microorganism which produces clavulanic acid, such as various microorganisms belonging to various *Streptomyces* strains such as *S*. *clavuligerus* NRRL 3585, *S*. *jumoninensis* NRRL 5741, *S*. *katsurahamanus* IFO 13716 and *Streptomyces* sp. P 6621 FERM P2804 e.g. as described in JP Kokai 80-162993. The resulting aqueous broth may be subjected to conventional purification and concentration processes, for example involving filtration and chromatographic purification, such as disclosed in GB 1508977 and JP Kokai 80-62993, before extraction of the aqueous solution with an organic solvent to yield a solution of crude clavulanic acid in the organic solvent.

GB 1508977 discloses inter alia that salts of clavulanic acid can be obtained by absorbing the clavulanate anion in filtered broth on to an anion exchange resin, eluting therefrom with an electrolyte, desalting the resulting solution, applying the desalted solution to a further anion exchange resin, chromatographically eluting therefrom with an electrolyte, desalting the resulting solution and thereafter removing the solvent. This process can be used to give acceptable yields of pure material but the use of resin columns involves significant investment and they can introduce limitations in large scale production operations. It would therefore be desirable to have an alternative procedure available that involved few resin utilizing stages.

GB 1543563 discloses a process for the preparation of clavulanic acid salts via precipitation of lithium clavulanate. GB 1578739 describes various amine salts of clavulanic acid as pharmaceutical compounds. EP 0026044 discloses the use of the tertiary-butylamine salt of clavulanic acid as a useful intermediate in the preparation of clavulanic acid. The salt has been disclosed in BE 862211, but only as a suitable ingredient for pharmaceutical formulations. PT.94.908 describes the use of tri-(lower alkyl)amine salts and the dimethylaniline salts of clavulanic acid in a purification process for clavulanic acid in which the triethylamine salt of clavulanic acid is formed and is then converted into a silyl diester of clavulanic acid. EP 0887178A discloses a process for the purification of clavulanic acid in which organic amines may be used to form an intermediate amine salt with clavulanic acid in an impure solution.

The present invention provides the use of the salt of clavulanic acid with an amine of formula (II): 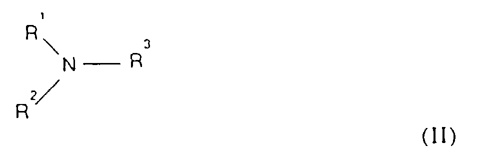 as an intermediate in a process for the preparation of clavulanic acid or pharmaceutically acceptable salts and esters thereof, wherein R¹, R² and R³ are selected according to the following options:
(1) R¹ being an optionally substituted cyclic group of general formula:

   R-(CHR⁴)ₘ-

   where m is zero or an integer 1 to 5, R is an optionally substituted aliphatic hydrocarbon ring system containing from 3 to 8 ring carbon atoms, R⁴ is hydrogen or alkyl, amino- or hydroxy- substituted alkyl or substituted amino- substituted alkyl, or a group of the same general formula or R¹ above:, R² and R³ may be selected from the same groups from which R¹ is selected, or from hydrogen, alkyl, alkenyl, amino-or hydroxy-substituted alkyl or alkenyl, or substituted amino-substituted alkyl or alkenyl; but with the exception of cyclohexylamine: or
(2) each of R¹, R² and R³ are the same or different and are independently selected from hydrogen, alkyl, alkenyl, amino - or hydroxy- or alkoxy- substituted alkyl or alkenyl, or substituted amino- substituted alkyl or alkenyl, but with the exception of t-butylamine, s-butylamine, N,N-dimethylethylamine, 1,2-dimethylpropylamine, neopentylamine and 2-amino-3,3-dimethylbutane; and provided that if the amine (II) is trimethylamine or triethylamine the salt of clavulanic acid with the amine (II) is formed by reaciton of clavulanic acid or a labile derivative thereof in solution in an organic solvent with the amine (II) or a labile derivative thereof, and the salt is then isolated as or in a separate phase from the organic solvent: or
(3) R¹ being an optionally substituted aryl group of general formula: 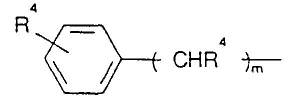 where R⁴ is hydrogen or one or more substituents, and m is zero or an integer 1 to 5, and R² and R³ are independently selected from hydrogen, alkyl, amino- or hydroxy-substituted alkyl or substituted - amino- substituted alkyl or groups of the same general formula as R¹, provided that if R⁴ is hydrogen and m is zero, then R² and R³ are not both methyl; but with the exception of benzylterbutylamine: or
(4) R¹ and R², and optionally R^{3,} together with the nitrogen atom shown being the residue of an optionally substituted heterocyclic ring system including the nitrogen atom as a ring member, and optionally including one or more additional ring hetero atoms, and if R³ is not part of the ring system it is independently selected from hydrogen, alkyl, amino- or hydroxy- substituted alkyl or substituted amino-substituted alkyl; but with the exception of piperidine: or
(5) R¹ being a group of general formula: 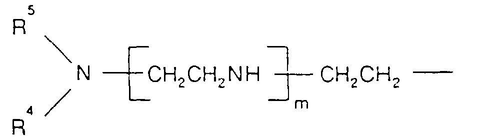 where R⁴ and R⁵ are independently hydrogen, alkyl, amino- substituted alkyl or substituted amino- substituted alkyl, and R² and R³ are independently selected from hydrogen, alkyl, amino- or hydroxy- substituted alkyl or substituted amino-substituted alkyl, and m is zero or an integer 1 to 5: or
(6) One or both of R¹ and R² are hydrogen and R³ represents the residue of an amino acid in which the carboxylate group of the amino acid may be esterified or in the form of an amide.

When alkyl groups or substituted alkyl groups are referred to herein unless otherwise defined herein they may suitably contain 1 to 6 carbon atoms in the alkyl system. Suitable substituents on amino groups include alkyl.

In option (1) above the amine (II) is suitably other than an amine in which R¹ is a cycloalkyl group and m is zero and R² and R³ are both selected from cycloalkyl or from hydrogen or CₙH₂ₙ₊₁ where n is 1 to 7.

In option (1) above the cyclic group R may suitably be saturated, with m being suitably zero. The group R may be monocyclic or polycyclic, and each ring may suitably contain 5, 6 or 7 ring carbon atoms including atoms shared between rings in fused or bridged ring systems. Suitably the cyclic group R may be unsubstituted.

Suitably the amine (II) may include two or more cyclic groups R¹ or a fused ring system R¹ or a substituted ring system R for example having one or more alkyl substituents such as methyl. Suitably R² and R³ may be other than hydrogen, eg one or both may be alkyl or substituted alkyl.

Examples of such amines include cyclopentylamine, cycloheptylamine, NN-dimethylcyclohexylamine, dicyclohexylamine, adamantylamine, NN-diethylcyclohexylamine, N-isopropylcyclohexylamine, N-methylcyclohexylamine, cyclopropylamine, cyclobutylamine, norbornylamine, and dehydroabietylamine.

In option (2) above the amine (II) is suitably other than an amine in which R¹ is hydrogen or CₙH₂ₙ₊₁ where n is 1 to 7 and R² and R³ are also both selected from hydrogen or CₙH₂ₙ₊₁ where n is 1 to 7.

Suitably in option (2) above, R¹ may be an alkyl or substituted alkyl group of general formula: 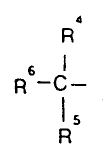 where R⁴, R⁵ and R⁶ independently represent C₁₋₁₀ alkyl, or amino- or hydroxy-substituted alkyl or substituted amino- substituted alkyl.

R⁴, R⁵ and R⁶ may suitably all be alkyl, suitably two of R⁴, R⁵ or R⁶ being methyl. Examples of such amines include t-octylamine, (ie 2-amino-2,4,4-trimethylpentane) and t-amylamine. Alternatively two of R⁴, R⁵ or R⁶ may be alkyl and one may be hydroxy- substituted alkyl. Examples of such amines include 1-hydroxy-2-methyl-2-propylamine.

In option (2) above, R¹ may alternatively suitably be C₁₋₂₀ alkyl, e.g. C₈₋₂₀ alkl, C₁₋₂₀ alkenyl, C₁₋₂₀ hydroxyalkyl, or C₁₋₂₀ aminoalkyl.

Examples of such amines include tri-n-propylamine, tri-n-octylamine, tri-n-butylamine, dimethylamine, i-propylamine, di-n-hexylamine, di-n-butylamine, diethylamine, 2-aminoethanol, NN-diethylethanolamine, NN-dimethylethanolamine, ethanolamine, n-butylamine, n-hexylamine, n-octadecylamine, N-ethylethanolamine, 1-hydroxyethylamine, diethanolamine, NN-dimethylethanolamine, N-ethyl diethanolamine, 1, 6-diamino hexane, triethanolamine, diisobutylamine, diisopropylamine, 2-methoxyethylamine, hydroxylamine, ammonia, methylamine, ethylamine, n-propylamine, n-butylamine, n-pentylamine, n-hexylamine, n-heptylamine, n-octylamine, n-nonylamine, n-decylamine, n-undecylamine, n-dodecylamine, n-prop-2-ylamine, n-but-2-ylamine, n-pent-2-ylamine, n-hex-2-ylamine, n-hept-2-ylamine, n-oct-2-ylamine, n-non-2-ylamine, n-dec-2-ylamine, n-undec-2-ylamine, n-dodec-2-ylamine, n-hex-3-ylamine, n-hept-3-ylamine, n-oct-3-ylamine, n-non-3-ylamine, n-dec-3-yl-amine, n-undec-3-ylamine, n-dodec-3-ylamine, n-oct-4-ylamine, n-non-4-ylamine, n-dec-4-ylamine, n-undec-4-ylamine, n-dodec-4-ylamine, n-non-5-ylamine, n-undec-5-ylamine, n-dodec-5-ylamine, and n-octadecylamine.

In option (3) above the amine (II) is suitably not an amine in which R² and R³ are selected from hydrogen or CₙH₂ₙ₊₁ where n is 1 to 7 or benzyl or substituted benzyl.

In option (3) above suitable substituent groups R⁴ include C₁₋₆ alkyl such as methyl, phenyl or optionally substituted phenyl, carboxylic or sulphonic acid groups and derivatives of such acid groups such as esters (eg C₁₋₆ alkyl esters) and amides; nitro, and halogen such as bromine. Suitably m may be zero, 1 or 2, and R⁵ may be hydrogen or methyl. Suitably R² and R³ may be hydrogen, or one of R² and R³ may be hydrogen and the other may be an aromatic group of the same general formula as R¹.

Examples of such amines include 1-phenylethylamine, p-toluidine, p-aminobenzoic acid, p-bromoaniline, ethyl-4-aminobenzoate (ie benzocaine), benzylamine, diphenylamine, p-methylaminobenzene sulphonamide, m-nitroaniline, N,N'-dibenzylethylenediamine (ie benzathine), diphenylmethylamine, 4-methylbenzylamine and 4-phenylbutylamine.

In option (4) above the ring system may be aromatic or aliphatic, and may be monocyclic or polycyclic. Suitably each ring in the system may contain 5 or 6 ring atoms, including the ring nitrogen atoms, and including atoms which are shared between rings. Suitable optional substituents on the ring system include alkyl, amino, substituted amino, oxo and halogen. If the ring system includes additional ring hetero atoms to the nitrogen atom shown, such hetero atoms may suitably be selected from nitrogen and oxygen.

Examples of classes of such amines include substituted piperidines and optionally substituted piperidines, for example where the substituents are selected from alkyl, hydroxyalkyl, halogen, amino, substituted amino and amino-substituted alkyl. Specific examples of such amines include N-ethyl piperidine, 2, 6-dimethyl piperidine, 2-methyl-N-hydroxypropyl piperidine (ie cyclo- methycane), 4-methyl piperazine, 1-methyl-4-phenyl piperazine, N-ethyl morpholamine, hexamethylenimine, pyridine, 2-propylpyridine, 3-chloro-2-aminopyridine, morpholamine, 1, 5-diazabicyclo [4,3,0] non-5-ene, 1, 4-diazabicyclo [2,2,2] octane, pyrrolidone, quinuclidine and xanthinol.

In option (5) above R⁴ and R⁵ may suitably both be hydrogen, or one may be hydrogen and the other alkyl. R² and R³ may suitably be hydrogen or alkyl.

Examples of such amines include ethylene diamine, NN-diethylethylene diamine, NN'-diisopropylethylenediamine and triethylene tetramine.

In option (6) above the amino acid may be a naturally occurring amino acid. Amino acid esters may be with alkyl groups or substituted alkyl groups such as benzyl.

Examples of such amines include arginine, ornithine, histidine, lysine, benzylglycine, 3-amino-3-methylbutanoic acid, L-ethyl lysinate, L-methyl histidinate, methyl N-carbobenzyloxy-L-lysinate, methyl L-phenylalanate, ethyl glycyl glycinate, ethyl p-hydroxy phenyl glycinate, ethyl p-hydroxy phenyl glycinate, ethyl glycinate, ethyl L-tyrosinate, p-methoxybenzyl α-aminophenylacetate, n-butyl α-aminophenylacetate, methyl arginate, benzylglycine, benzyl phenylglycine, 1-nitrobenzyl phenyl glycine, n-butyl phenylglycine, p-methoxybenzyl phenylglycine, ethyl phenyl glycine, p-nitrobenzyl p-hydroxyphenyl-glycine, p-nitrobenzylserine, n-butyl serine, methyl arginine, dimethyl glutamate, p-nitrobenzyl tyrosinate, p-nitrobenzyl glycinate, benzylglycinate, p-nitrobenzyl α-amino-p-hydroxy-phenyl acetate, p-nitrobenzyl α-aminophenylacetate, ethyl α-amino-p-hydroxy phenyl acetate, ethyl L-tyrosinate.

When the amine (II) contains more than one nitrogen atom the clavulanic acid may form a salt with one or more of the nitrogen atoms, for example as in NN'-diisopropylethylenediamine diclavulanate.

Of the amines mentioned above, preferred amines are: phenylethylamine, t-amylamine, t-octylamine, 1-hydroxy-2-methyl-2-propylamine, cyclopentylamine, cycloheptylamine, 1-adamantanamine, N-ethylpiperidine, N'N'-diisopropylethylenediamine and N N-dimethylcyclohexylamine.

In a further aspect the present invention provides a process for the preparation and/or purification of clavulanic acid or a pharmaceutically acceptable salt or ester thereof which process comprises:
i) contacting impure clavulanic acid or a labile derivative thereof in solution in an organic solvent, with an amine of the formula (II) or a labile derivative thereof,
ii) isolating the salt of clavulanic acid formed with the amine of formula (II), and,
iii) converting the thus formed salt into clavulanic acid or a pharmaceutically acceptable salt or ester thereof.

In the process of the present invention the salt of clavulanic acid with the amine (II) may be used to purify impure clavulanic acid during its preparation. Therefore the salt is may be formed in a solution of clavulanic acid or a labile derivative thereof containing impurities, isolating the salt as a separate phase, eg as a solid precipitate, from the solution containing residual impurities, then reforming clavulanic acid or forming a pharmaceutically acceptable salt or ester thereof.

Suitable labile derivatives of clavulanic acid include salts, eg an alkali metal salt such as lithium or sodium clavulanate or esters, such as silyl esters. Suitable labile derivatives of the amine (II) include salts such as the phosphate, borate, chloride, chlorate, perchlorate, bromide, toluene sulphonate or alkanoates, such as the acetate or ethylhexonoate. Conveniently the amine (II) itself is contacted with impure clavulanic acid itself in solution in an organic solvent.

The above process is suitably carried out in an organic solvent, which although preferably substantially dry, for example containing less than 6 g/L, eg 0.25-0.6 g/L of water, may contain some water, as a solvent for the clavulanic acid and the amine (II). A suitable degree of dryness may be achieved by conventional dewatering processes such as centrifuging. Water present in the solvent may be dissolved or in the form of droplets of a separate phase.

The solution of clavulanic acid in organic solvent may be obtained by extraction of an acidified aqueous solution of clavulanic acid such as the fermentation liquor referred to above. If the initial source of the clavulanic acid is a broth resulting from fermentation of a clavulanic acid-producing microorganism, such as those mentioned above, then to obtain a solvent extract of a suitable concentration of clavulanic acid for use in this process it may be desirable not to extract the broth itself, but to at least remove some of the suspended solids in the broth, e.g by filtration prior to extraction. It may also be desirable in addition to pre-concentrate the aqueous solution of clavulanic acid obtained in fermentation, so that for example the aqueous solution of clavulanic acid is several times more concentrated in clavulanic acid than the starting broth, for example pre-concentrated to a concentration of ca. 10 - 100 mg/ml. e.g 10 - 40 mg/ml, such as 10 - 25 g/L clavulanic acid.

Suitable pre-concentration processes include absorption of the clavulanic acid onto an anion exchange resin, followed by elution of the clavulanic acid therefrom with an aqueous solution of an electrolyte such as sodium chloride, and optionally desalting. It is also preferred to acidify the aqueous solution, e.g the broth or the preconcentrated aqueous solution prior to solvent extraction, e.g to pH 1 to 3, e.g around pH 1.5 to 2.5. It is also preferred to dry or de-water the organic solvent extract prior to formation of the salt with the amine (II), e.g to less than 6g/L of water. Preferably the extraction is carried out at a temperature from 5 to 15^{o}C.

Suitable organic solvents in which impure clavulanic acid may be contacted with the amine (II) include hydrocarbon solvents such as toluene and hexane, ethers such as tetrahydrofuran, dioxan, diethyl ether, halogenated solvents such as dichloromethane and chloroform, ketones such as acetone and methyl isobutyl ketone, and esters such as ethyl acetate. Solvents which include a carbonyl group, eg those of the formula (III): 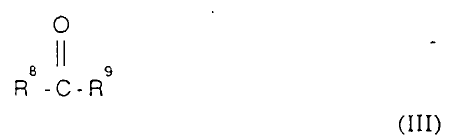 wherein R⁸ is a C₁₋₆ alkyl group or a C₁₋₆ alkoxy group and R⁹ is a C₁₋₆ alkyl group are examples of a sub-class of suitable solvents, for example organic ketones or organic alkanoate esters. The present invention also encompasses the use of mixtures of such solvents.

More suitably the organic solvent is one which can be used directly to extract the acidified aqueous for example organic alkyl alkanoate esters, ketones and certain aliphatic alcohols, or mixtures thereof, such as ethyl acetate, methyl acetate, propyl acetate, n-butyl acetate, methyl ethyl ketone, methyl isobutyl ketone, tetrahydrofuran, butanol and mixtures of such solvents. Of these the most suitable appear to be methyl isobutyl ketone, methyl ethyl ketone, and ethyl acetate. Suitable solvent mixtures include methyl ethyl ketone/methyl isobutyl ketone and tetrahydrofuran/methyl isobutyl ketone. A preferred solvent is ethyl acetate.

Suitable solvents for the amine (II) include those referred to above in which the clavulanic acid may be dissolved, or extracted, for example acetone, ethyl acetate, methyl isobutyl ketone, and methyl ethyl ketone.

It appears to be particularly desirable to include ketones such as acetone in the solvent system, as these appear to inhibit the formation of the salt of clavulanic acid with the amine (II) as an oil.

In general one equivalent of the amine (II) or a slight excess thereof per mole of clavulanic acid is used to produce the salt of clavulanic acid. Solutions of clavulanic acid and amine (II) may for example be mixed slowly with stirring and the mixture stirred for some time after addition is complete. The reaction between the clavulanic acid or its labile derivative is suitably carried out at a temperature below ambient, for example 0 to 15°C, eg 0 to 10°C, eg 0 to 5°C. A suitable concentration for the clavulanic acid or its labile derivtive in the solution is at least 1.0 g/L, for example in the range 1.0 to 4.0 g/L of clavulanic acid. It may be advantageous to further concentrate the solvent extract to a concentration in excess of this eg greater than 20g/L.

For example in another procedure the amine (II) may be introduced by mixing it into a stream of a solution of the clavulanic acid in the solvent, so that the salt is formed in the stream, either in solution or as particles or suspended droplets of the dissolved salt in suspension. The amine (II) introduced in this way may be introduced neat, or may be introduced as a solution in a solvent, for example the same organic solvent as the clavulanic acid is dissolved in.

The desired salt of clavulanic acid with the amine (II) may then be isolated. In this way, the salt of clavulanic acid with the amine (II) is separated from most or all of the impurities. Isolation may be effected in a conventional manner, for example by centrifugation or filtration.

In an alternative isolation procedure the salt of clavulanic acid with the amine (II) may be isolated from the organic solvent, if the solvent is wholly or partly immiscible with water, by contacting the solvent with water so as to extract the salt, which may be in solution or suspension, from the organic solvent and to form an aqueous solution of the salt. As salts of clavulanic acid with the amine (II) are fairly soluble in water, such an aqueous solution may be very concentrated, eg around 20-30% w:w.

In this way the bulk of organic impurities in the organic solvent solution of clavulanic acid remain in the organic solvent whilst the clavulanic acid, in the form of its salt with the amine (II), in a relatively pure state is obtained in the aqueous solution. The aqueous solution of the clavulanic acid salt so formed may be subjected to conventional further treatment, eg purification, or conversion into clavulanic acid or a pharmaceutically acceptable salt or ester as described below.

In another alternative or additional procedure the clavulanic acid and the amine may be mixed in solution in a first organic solvent, then the salt may be caused to separate from solution by addition of a second organic solvent. Suitably the first organic solvent may be an organic ester such as ethyl acetate, and the second solvent may for example be a halogenated solvent such as chloroform, an ether such as diethyl ether, a hydrocarbon such as toluene, an alcohol such as ethanol, or a solvent of formula (III) above such as acetone or methyl isobutyl ketone.

Some of the above-mentioned salts of clavulanic acid are believed to be novel compounds and as such are a further aspect of this invention, for example the salts of clavulanic acid with phenylethylamine, t-amylamine, 1-hydroxy-2-methyl-2-propylamine, cyclopentylamine, cyclohexylamine, 1-adamantanamine. N-ethylpiperidine, NN-dimethylcyclohexylamine and NN'-diisopropylethylenediamine.

In one embodiment of this invention, the salt of clavulanic acid with the amine (II) may be employed as an acetone solvate. Acetone solvates in some cases have advantageous stability and purity characteristics compared to salts of clavulanic acid with amines themselves. Such solvates are particularly useful in the present invention because they can often be isolated as a highly pure and stable crystalline compound.

Accordingly the present invention also provides the salt of clavulanic acid with the amine (II) in the form of an acetone solvate. During isolation and/or drying, some acetone may be lost since the strength of solvation may not be high, but the amount of acetone in the product is not critical.

The acetone solvate may be formed by contacting clavulanic acid with the amine (II) in the presence of acetone. In general, a solution containing clavulanic acid may be mixed with at least the same volume of acetone together with the amine (II), when the salt is precipitated.

The amine (II) may be dissolved in acetone and mixed with a solution of clavulanic acid in an organic solvent. Favoured organic solvents include ethyl acetate, tetrahydrofuran, methyl ethyl ketone, methyl isobutyl ketone and mixtures of such solvents, of which ethyl acetate is preferred. Alternatively an aqueous solution of the salt of clavulanic acid with the amine (II), obtained by water extraction as outlined above, may be mixed with acetone to form the solvate. Suitably a concentrated aqueous solution of the salt may be mixed with excess acetone to form the solvate.

Recrystallisation of the salt of clavulanic acid or the acetone solvate may be advantageous to further reduce the level of impurities. A convenient solvent for the recystallisation is aqueous acetone. Such recrystallisation is performed in a conventional manner for example the salt or solvate is dissolved in water, treated with a small amount of acetone, filtered, and then treated with larger volumes of acetone optionally with stirring and/or cooling to afford the recrystallised product.

In another aspect the present invention provides a process for the preparation of clavulanic acid or a pharmaceutically acceptable salt or ester thereof which process comprises converting the salt of clavulanic acid with an amine of formula (II) into clavulanic acid or a pharmaceutically acceptable salt or ester thereof.

The pharmaceutically acceptable salts and esters of clavulanic acid prepared by the processes of this invention include those described in GB 1508977 and 1508978 which are herein incorporated by reference.

Particularly suitable pharmaceutically acceptable salts include the pharmaceutically acceptable alkali and alkaline earth metal salts, for example the sodium, potassium, calcium and magnesium salts. Of these salts the sodium and potassium are most suitable and the potassium is preferred.

Suitable esters include those cleavable to provide clavulanic acid or a salt thereof, by chemical methods such as hydrogenolysis or by biological methods.

The salt of clavulanic acid with amine (II) optionally in the form of its acetone solvate may be converted into clavulanic acid or a pharmaceutically acceptable salt or ester thereof by for example ion-replacement in the case of the free acid or salts, or by esterification.

Ion-replacement may be performed using ion-exchange resins, for example by passing a solution of the salt through a bed of a cation exchange resin in sodium, potassium or calcium form. Suitable cation exchange resins include Amberlite IR 120 and equivalent resins.

Alternatively ion-replacement may be effected by reaction of the protonated amine cation with a salt-precusor compound, which may be a base, for example a carbonate, bicarbonate or hydroxide of a pharmaceutically acceptable alkali or alkaline earth metal, or a salt of an organic carboxylic acid with a pharmaceutically acceptable cation such as an alkali or alkaline earth metal, for example a salt of an alkanoic acid of formula (IV):

R¹⁰-CO₂H (IV)

wherein R¹⁰ is an alkyl group, containing for example from 1 to 20 carbon atoms, preferably from 1 to 8 carbon atoms. Examples of suitable salts include the acetate, propionate or ethylhexanoate salts, potassium 2-ethylhexanoate and sodium 2-ethylhexanoate being preferred. Typically the salt of clavulanic acid with amine (II) in solution may be reacted with a salt of an alkali metal with acid (IV) in solution or suspension in a suitable solvent, which may for example be an organic solvent, water, or a mixture of water and an organic solvent such as isopropanol. Suitably solutions of the salt of clavulanic acid with the amine (II) and of the salt-precurssor compound may be mixed, and the pharmaceutically acceptable salt allowed to crystallise. Suitably the reaction may be carried out of a temperature below ambient, e.g. 0 to 15° C, e.g. 0 to 10°C, suitably 0 to 0.5°C. Suitably if the salt of clavulanic acid with the amine (II) is formed an aqueous solution it may be precipitated out by admixing the aqueous solution with excess acetone.

Suitable methods of esterification include:
a) the reaction of the salt of clavulanic acid with the amine (II) with a compound of the formula Q-R¹¹ wherein Q is a readily displaceable group and R¹¹ is an organic group;
b) the reaction of the salt of clavulanic acid with the amine (II) with an alcohol or thiol in the presence of a condensation promoting agent such as carbodiimide; and
c) the reaction of the salt of clavulanic acid with amine (II) with a diazo compound.

The foregoing processes extend to cover those aspects wherein the salt of clavulanic acid with amine (II) is first converted to clavulanic acid or another salt thereof and subsequently is converted to the desired ester. Further details of esterification methods are disclosed in GB 1508977 and 1508978. Use of the present invention enables salts and esters of clavulanic acid to be more readily obtained in pure form than operation of the processes of GB1508977 and 1543563.

The invention will now be described by way of example only.

### Example 1

In each of the following the procedure was the same the amine was mixed with clavulanic acid in solution in THF and rapid crystallisation to form a solid salt was observed.

| **Amine** | **Comments** | **Stability of Salt** |
|---|---|---|
| D-(+)-α-phenylethylamine | crystalline solid | Stable >70h, 20°C 50% RH |
| t-octylamine | crystalline solid | Stable >70h, 20°C 50% RH |
| 1-hydroxy-2-methyl-2-propylamine | crystalline salt | |
| cyclopentylamine | crystalline salt | 50% RH, 20°C darkens after 72h |
| cycloheptylamine | crystalline salt | 50% RH, 20°C darkens after 72h |
| 1-adamantanamine | crystalline salt | 50% RH, 20°C stable >70h. |
| N-ethylpiperidine | crystalline salt | 50% RH, 20°C stable >70h. |
| N,N-dimethylcyclohexylamine | crystalline salt | 50% RH, 20°C stable >90h. |
| ammonia | crystalline salt | >70h, 50% RH some discolouration |
| xanthinol | white solid | hygroscopic |
| triethylene tetramine | pale yellow solid | low assay |
| p-methoxybenzyl-α-aminophenylacetate | solid salt | white after 16h dry atmos. room temperature., yellow after 16h 50% RH room temperature. |
| n-butyl α-aminophenylacetate | solid salt | Yellow after 16h dry atmos. room temperature.,orange after 16h 50% RH room temperature. |
| methyl arginate | solid salt | white after 16h dry atmos. 100m temp., orange after 16h 50% RH room temp. |

### Example 2

In the following a salt of clavulanic acid with an amine was formed in solution in a first organic solvent, and was then caused to separate out from solution as a solid precipitate by mixing with a second organic solvent.
**2a. t-Octylamine** A stock solution of clavulanic acid in ethyl acetate was prepared containing ca. 28g/L of clavulanic acid in an impure form, by extraction of an impure *S*. *clavuligerus* fermentation broth with ethyl acetate. To 46 ml of this was added t-octylamine (0.84g). After 10 minutes the mixture became cloudy and fine crystals of the salt crystallised out. To an aliquot of the solution was added chloroform, resulting in the formation of larger needles. To a further aliquot was added acetone, again resulting in the formation of needle crystals, but smaller and less quickly than with chloroform. To the remainder of the solution was added ca. 20 ml chloroform then a volume of toluene approximately equal to the initial bulk of the solution, which resulted in precipitation of a substantial quantity of needle crystals.
**2b. Dicyclohexylamine** - To 46 ml of the stock solution prepared as for 2a above was added dicyclohexylamine (1.18g). This resulted in a clear solution. Acetone was added to one aliquot, forming a fine amorphous deposit.
**2c. Ammonia** - To 500 ml of the stock solution prepared as for 2a above was added ammonia (21.1ml of 2.5M ammonium 2-ethylexanoate in ethyl acetate, ie ca. 1 equivalent). Addition of 50 ml of industrial methylated spirit (IMS) resulted in the ready formation of a precipitate of fine needles. It was noted that when an impure solution of clavulanic acid was used, as manifested by the colour of the solution, a considerable amount of the coloured impurities were left in solution. Yield - 75%.
**2d. N,N-Dimethylcyclohexylamine** - To 500 ml of the stock solution prepared as for 2a above was added N,N-dimethylcyclohexylamine (6.7 ml). An oil formed on commencement of addition. Acetone (150 ml) was gradually added, resulting in a cloudy solution. An aliquot of this cloudy solution was taken, and to it was added diethyl ether, resulting in crystallisation. Diethyl ether (100 ml) was added to the main bulk of the solution resulting in an immediate crystallisation. The crystals (13.4g) were filtered and washed with acetone.
**2e. t-Octylamine** - To 500 ml of the stock solution prepared as for 2a above was added t-octylamine (6.7g). The solution became slightly hazy. Acetone (20 ml) was added, which cleared the solution. To an aliquot of the solution was added diethyl ether, resulting in immediate crystallisation. Diethyl ether (55 ml) was added to the main bulk of the solution, resulting in crystallisation. The crystals were filtered and washed with acetone. The yield (12.9g) represented a 77% recovery of the clavulanic acid from the solution.
**2f. Benzathine** - Benzathine diacetate (9.16g) was stirred with aqueous sodium hydroxide (5N, 10.15 ml). The aqueous solution was extracted with ethyl acetate (55 ml), and the extract was added to the stock solution of clavulanic acid prepared as for 2a above (1.0L). An oil formed initially. Acetone (600 ml) was added, and crystals formed (About 10 ml of diethyl ether was added being a residue of a test tube trial). Methyl isobutyl ketone (200 ml) was then added, and the mixture was then made up to 2L with acetone. The crystals formed were filtered and washed with acetone. The yield was 11.5g representing 59.1 % recovery of the clavulanic acid.

### Example 3

A solution of clavulanic acid in ethyl acetate (ca 20µg/ml) was diluted with an equal volume of acetone. An acetone solution of t-octylamine (1.25 mole equivalents) was then added dropwise over ½ hours at 10°C. After further stirring for 1 hour the precipitated crystals were collected, washed with acetone and dried under vacuum.

A precipitate formed quite readily and was white in colour. Yield (corrected for purity) = 76%.

### Example 4

An 0.012M solution of benzyl clavulanate in THF was catalytically hydrogenated to form a solution of clavulanic acid in THF. To this solution (100 ml) was added 1-aminoadamantane (25 ml 0.012M in THF) at ambient temperature. Rapid crystallisation occurred. The solution was stirred at ambient temperature for 30 minutes, then at 5°C for two hours. The solution was then filtered, and the crystals washed and dried. Yield = 83%. Melting point of the salt was 190-192°C (d).

### Example 5

Benzyl clavulanate (purified by Sephadex chromatography, 20g, 0.07 moles) was dissolved in tetrahydrofuran (distilled from calcium hydride 400 ml) and 10% palladium on charcoal catalyst (5.7g) added. The mixture was hydrogenated with stirring at ambient temperature and about 15 psi for 20 - 30 minutes. The state of reaction was judged by thin layer chromatography using silica plates developed with ethyl acetate and visualised using triphenyltetrazolium chloride spray reagent. Clavulanic acid Rf 0.0, benzyl ester 0.4.

The reaction mixture was filtered and the filter pad well washed. The combined filtrates (∼500ml) containing clavulanic acid were treated with stirring with 2-amino-2,4,4-trimethylpentane (9.0g, 0.07 moles) in dry tetrahydrofuran (50 ml). Crystallisation was observed within one minute. The mixture was stirred or 0.5 hours at ambient temperature and then 2 hours at 5°C. The product was filtered off, was filtered off, washed with dry tetrahydrofuran (100 ml) and dried in vacuo for 12 hours to afford 23.0g, 100% of the title salt, having m.p. 160 - 170°(d).

### Example 6

The preparation was carried out as in Example 5 using benzyl clavulanate (0.9g, 0.003 moles) and treating the resulting clavulanic acid solution with 2-amino-2-methylpropane (0.22g, 0.003 mole) in dry tetrahydrofuran (10 ml). The title salt 0.6g, 73% yield, had m.p. 150-152°(d).

### Example 7

The preparation was carried out as in Example 5 using benzyl clavulanate (0.9g, 0.003 moles) and treating the resulting clavulanic acid solution with D(+) 1-methylbenzylamine in dry tetrahydrofuran (10 ml). The mixture was stored at 5° for two days during which time a slow crystallization occurred. Filtration afforded the title salt 0.6g, 62% yield, having m.p. 125°(d).

### Example 8

An aqueous solution of lithium clavulanate was mixed with an aqueous solution of an alkylammonium phosphate. The precipitated lithium phosphate was filtered off, and the amine clavulanate salt was precipitated by addition of acetone. It was found that sodium clavulanate could also be used, but that there was then a need to add ethanol to encourage separation of sodium phosphate. Also amine hydrochlorides could be used.

In this way lithium clavulanate was used to prepare N-ethylpiperidine clavulanate, and sodium clavulanate was used to prepare N-ethylpiperidine clavulanate.

### Example 9

An impure solution of clavulanic acid being a crude extract from an *S*. *clavuligerus* fermentation broth after some pre-purification by ion-exchange (500 ml, 21µg/ml in ethyl acetate) was mixed with acetone (150 ml), and treated gradually with a solution of N,N-dimethylcyclohexyl amine (6.7 ml) in acetone (25 ml). An aliquot was removed and treated with diethyl ether and immediate crystallisation occurred. Seeding the main bulk this led to rapid crystallisation of white platalets which was made more complete by the addition of ether (110 ml). The amine salt was filtered off and washed with acetone (3 x 100 ml) and air dried. Yield = 13.4g, 82% recovery, 64.9% pfa (theoretical 61.0%).

### Example 10

To the impure solution of clavulanic acid used in example 9 (500 ml, 21µg/ml) additionally containing acetone (20 ml) was added t-octylamine (7.6g, 1.0 e.g.) which produced a slight haziness. Addition of diethyl ether (55 ml) caused the separation of the amine salt as fine white needles which were filtered off and washed with acetone. Yield 12.9g, 77.2% recovery, 62.8% pfa (theoretical = 60.6%).

### Example 11

An impure solution of clavulanic acid in ethyl acetate (1L, 10.14 µg/ml) obtained by extraction of an *S*. *clavuligerus* fermentation broth with ethyl acetate and some pre-purification by ion-exchange, was mixed with acetone (600ml), and then treated with benzathine (6.15g) in ethyl acetate (55 ml). The mixture started to crystallise as the addition of benzathine neared completion. Methyl isobutyl ketone (200 ml) was then added to make precipitation more complete. The crystals were filtered off and was washed with acetone (3 x 100 ml). Yield = 11.5g, 67.0% recover.

### Example 12.

The procedure of Example 11 was repeated using the impure, wet (ca. 1% water), solution of clavulanic acid in ethyl acetate. To separate 1L batches of this solution was added with stirring an excess of neat ethylene diamine, NN'-diethylethylene diamine, and NN'-diisopropylethylenediamine, the quantity of each amine being in excess of the amount needed to form a diammonium salt with the clavulanic acid. After continued stirring a precipitate of the salt formed. A further crop of crystals was obtained by addition of excess acetone. The precipitated diammonium salt was filtered off and washed with acetone.

The crystals of each of these diammonium salts so formed were converted to potassium clavulanate by dissolution of the salt in the minimum quantity of water, followed by the addition of a solution of an excess of potassium 2-ethylhexanoate in isopropyl alcohol. After continued stirring a precipitate of potassium clavulanate formed and was filtered, washed with isopropyl alcohol and dried.

### Example 13

A solution of clavulanic acid (0.0046 mole) in tetrahydrofuran (30 ml) was treated with a solution of trimethylamine in methanol (1.0 ml of a 25% w/v solution, ca. 0.0042 mole), giving a pale yellow solution. Crystallization was induced by seeding and standing at room temperature. Crystallization was rapid, giving fine needles, and was completed by standing overnight at +4°. The product was filtered off, washed with a small volume of fresh solvent (ca. 5 ml) and dried in vacuo. Yield 0.9 g (75%), m.p. >310°, slowly turning brown above ca. 130°.

| | | | | |
|---|---|---|---|---|
| Assay B 24641: | Found | C, 51.32; | H, 7.15; | N, 10.68% |
| C₁₁H₁₈N₂O₅ | requires | C, 51.16; | H, 7.02; | N, 10.84% |

| | | |
|---|---|---|
| Assay A 15230: | Imidazole | 77.2; 76.4% (calc. 77.1%, ca. 99.5% purity) |
| | Water | 1.18; 1.34% |

The compound (as cream coloured fine needles) deteriorates on standing in air over several days, picking up water and slowly turning yellow.

### Example 14

Two preparations of the above compound are included as each gave a slightly different result. The first gave a modest yield of a product containing water, to the extent of corresponding to a hemihydrate, while the second gave an essentially anhydrous material albeit in low yield.
**14A.** Clavulanic acid (0.00346 mole) in tetrahydrofuran (25 ml) was treated with triethylamine (0.4g, 0.00346 mole) and mixed to give a homogeneous clear solution. Crystallization was initiated by seeding and completed by standing overnight at +4°. The product was filtered off and dried in vacuo. Yield 0.7g (63%), as large off-white needles.

| | | | | |
|---|---|---|---|---|
| Assay B 24639: | Found | C, 54.74; | H, 7.95; | N, 8.94% |
| C₁₄H₂₄N₂O₅ | requires | C, 56.05; | H, 8.00; | N, 9.33% |
| C₁₄H₂₄N₂O_{5½}H₂O | requires | C, 54.36; | H, 8.14; | N, 9.05% |

| | | |
|---|---|---|
| Assay A 15220: | Imidazole | 61.0; 61.1% (calc. 64.4%, ca. 95.0% purity) |
| | Water | 2.64% (hemihydrate requires 12.91% |

**14B.** Clavulanic acid (0.021 mole) in tetrahydrofuran (60 ml) was treated with triethylamine (2.1g, 0.021 mole) and mixed resulting clear solution seeded with authentic salt. The product began crystallizing as long needles at room temperature, the process being completed at +4° overnight. The product was collected by filtratic, washed on the filter with fresh solvent (ca. 10ml) and diethyl ether (ca. 20 ml), and finally dried in vacuo, to give an off-white crystalline product. Yield 1.7g (27%).

| | | | | |
|---|---|---|---|---|
| Assay B 24676: | Found | C, 56.07; | H, 7.83; | N, 9.32% |
| C₁₄H₂₄N₂O₅ | requires | C, 56.05; | H, 8.00; | N, 9.33% |
| C₁₄H₂₄N₂O_{5½}H₂O | requires | C, 54.36; | H, 8.14; | N, 9.05% |

| | | |
|---|---|---|
| Assay A 15268: | Imidazole | 65.1; 65.1% (calc. 66.3%, ca. 98.2% purity) |
| | Water | 0.9% |
| | [α]_{D}²⁴ | +47.0° (c=0.2% in water) |

Like the trimethylamine salt the product deteriorates on standing in air over several days.

These two examples illustrate the formation of high purity clavulanic acid salts from solutions of clavulanic acid in organic solvents.

### Example 15

Separate samples of trimethylamine clavulanate and triethylamine clavulanate from examples 13 and 14 are dissolved in the minimum amount of cold water. To these solutions is added a strong solution of potassium 2-ethylhexanoate in isopropanol, with stirring. After continued stirring crystals of potassium clavulanate separate and may be filtered off, washed with cold isopropanol, and dried.

### Example 16

A impure aqueous solution of clavulanic acid, being obtianed from an *S*.*clavuligerus* fermentation broth by pre-purification involving ion-exchange chromatography or generally outlined above, containing ca. 17.1 g/L was acidified to pH 2.0 with 25% v/vsulphuric acid, and was then continuously extracted into ethyl acetate. The ethyl acetate extract was cooled to 2^{o}C, dewatered by centrifuging then dried with MgSO₄ then passed down a CPG carbon column. At this stage the ethyl acetate extract contained 6.02 g/L of clavulanic acid, and it was then concentrated by evaporation to a concentration of a 25.7 g/L of clavulanic acid, and was used at this concentration. The moisture level of the concentrate was ca. 026% v/v.

7.8 ml of t-octylamine was mixed with 25 ml of fresh ethyl acetate. This mixture was slowly added to 2 litres of the clavulanate rich ethyl acetate extract diluted back to a titre of 23.0 g/L with fresh ethyl acetate, with rapid stirring. The slurry was stirred for a further hour at 5^{o}C. The t-octylamine clavulanate was subsequently isolated by filtration, and wasshed with ethyl acetate. Final drying was carried out overnight in a vacuum oven at 20°C, with a nitrogen bleed. Product weight = 6.13 g.

### Example 17

5g of the amine salt produced in example 16 was dissolved in 97 ml of isopropanol. The product did not dissolve readily, and it was found necessary to apply gentle warming, to 24° C, in order to effect total dissolution. 14 ml of 1.5 N potassium 2-ethylhexanoate in isopropanol were added over 10 minutes. The slurry was subsequently stirred for 1.5 hours, at 5°C. The product potassium clavulanate was then filtered off, washed with small amounts of isopropanol then acetone, and dried under vcacuum overnight with a nitrogen bleed, at 20^{o}C. Product weight = 3.16g.

### Example 18

Filtered (RVF) *S*. *clavuligerus* fermentation broth containing 2 g/L at clavulanic acid was acidified to pH 1.6 with 25% v/v sulphuric acid and continuously extracted into ethyl acetate. The solvent extract was cooled to 3^{o}C then dewatered by centrifuging, then dried with MgSO₄, then passed down a CPG carbon column. The carbon-treated extract was then concentrated by evaporation to a concentration of clavulanic acid of ca. 20 g/L, with a moisture content of ca. 0.06% v/v.

13.5ml of t-octylamine was mixed with 43 mls of fresh ethyl acetate. This mixture was slowly added to 400 ml of clavulanate rich ethyl acetate extract at a titre of 20 g/L clavulanic acid, with rapid stirring. The slurry was stirred for a further hour at 5^{o}C. The t-octylamine clavulanate was subsequently isolated by filtration, and washed with ethyl acetate. Final drying was carried out overnight in a vacuum oven at 20^{o}C, with a nitrogen bleed. Product weight = 12.44 g.

### Example 19

11g of the amine salt produced in example 18 was dissolved in 213 mls of isopropanol. The product did not dissolve readily, even after applying gentle warming, to 24^{o}C. Water (3.75ml) was added to enable dissolution. Once a solution was obtained additional isopropanol (100ml) was added to reduce the water content prior to crystallisation. 30ml of 1.5 N. potassium 2-ethyl hexanoate in isopropanol was added over 15 minutes. The slurry was subsequently stirred for 1.5 hours at 5^{o}C. The potassium clavulanate product was then filtered off, washed with small amounts of isopropanol then acetone, and dried under vacuum overnight, with a nitrogen bleed, at 20^{o}C. Product weight = 7.29 g.

## Claims

1. A process for the preparation and/or purification of clavulanic acid or a pharmaceutically acceptable salt thereof which process comprises:
i)
a) obtaining impure clavulanic acid in a broth resulting from fermentation of a clavulanic acid - producing microorganism
b) contacting impure clavulanic acid or an alkali metal salt thereof in an organic solvent or solvents, with tertiary octylamine;
ii) isolating the tertiary-octylamine salt of clavulanic acid formed;
iii) converting the thus formed salt into clavulanic acid or a pharmaceutically acceptable salt or ester thereof characterised in that the aqueous solution of clavulanic acid obtained in the fermentation is preconcentrated before extraction.

2. A process according to claim 1 in which the aqueous solution of clavulanic acid is preconcentrated to a concentration of 10-100mg/ml.

3. A process according to claim 1 in which the aqueous solution of clavulanic acid is preconcentrated to a concentration of 10-40mg/ml.

4. A process according to claim 1 in which the aqueous solution of clavulanic acid is preconcentrated to a concentration of 10-25mg/ml.

5. A process according to any one of claims 1 to 4 in which the preconcentration process is by absorption of the clavulanic acid onto an ion-exchange resin followed by elution of the clavulanic acid therefrom with an aqueous solution of an electrolyte.

6. A process according to claim 5 in which the electrolyte is sodium chloride.

7. A process according to claim 5 or 6 in which the resulting concentrate is de-salted.

8. A process according to any one of claims 1 to 7 in which at least some of the suspended solids in the broth are removed by filtration.

9. A process for the preparation and/or purification of clavulanic acid or a pharmaceutically acceptable salt thereof which process comprises
i)
a) obtaining impure clavulanic acid in a broth resulting from fermentation of a clavulanic acid - producing microorganism
b) contacting impure clavulanic acid or an alkali metal salt thereof in an organic solvent or solvents, with tertiary octylamine;
ii) isolating the tertiary-octylamine salt of clavulanic acid formed;
iii) converting the thus formed salt into clavulanic acid or a pharmaceutically acceptable salt or ester thereof characterised in that the tertiary octylamine salt is caused to separate from solution by addition of a second organic solvent

10. A process according to claim 12 in which the first organic solvent is an organic ester and the second organic solvent is a halogenated solvent, an ether, a hydrocarbon, an alcohol or a solvent of formula (III):
R⁸-CO-R⁹ (III)
where R⁸ is a C₁₋₆alkyl group or a C₁₋₆alkoxy group and R⁹ is a C₁₋₆alkyl group.

11. A process according to claim 13 in which the first organic solvent is an ethyl acetate and the second organic solvent is chloroform, toluene, ethanol, acetone or methyl isobutyl ketone.

12. A process for the preparation and/or purification of clavulanic acid or a pharmaceutically acceptable salt thereof which process comprises
i)
a) obtaining impure clavulanic acid in a broth resulting from fermentation of a clavulanic acid - producing microorganism
b) contacting impure clavulanic acid or an alkali metal salt thereof in an organic solvent or solvents, with tertiary octylamine;
ii) isolating the tertiary-octylamine salt of clavulanic acid formed;
iii) converting the thus formed salt into clavulanic acid or a pharmaceutically acceptable salt or ester thereof characterised in that the extraction is carried out at a temperature of from 5 to 15°C.

13. A process for the preparation and/or purification of clavulanic acid or a pharmaceutically acceptable salt thereof which process comprises
i)
a) obtaining impure clavulanic acid in a broth resulting from fermentation of a clavulanic acid - producing microorganism
b) contacting impure clavulanic acid or an alkali metal salt thereof in an organic solvent or solvents, with tertiary octylamine;
ii) isolating the tertiary-octylamine salt of clavulanic acid formed;
iii) converting the thus formed salt into clavulanic acid or a pharmaceutically acceptable salt or ester thereof characterised in that the tertiary octylamine is introduced by mixing it into a stream of the clavulanic acid in the organic solvent or solvents.

14. A process for the preparation and/or purification of clavulanic acid or a pharmaceutically acceptable salt thereof which process comprises
i)
a) obtaining impure clavulanic acid in a broth resulting from fermentation of a clavulanic acid - producing microorganism
b) contacting impure clavulanic acid or an alkali metal salt thereof in an organic solvent or solvents, with tertiary octylamine;
ii) isolating the tertiary-octylamine salt of clavulanic acid formed;
iii) converting the thus formed salt into clavulanic acid or a pharmaceutically acceptable salt or ester thereof characterised in that the organic solvent containing the clavulanic acid is dewatered by centrifuging.

15. A process for the preparation and/or purification of clavulanic acid or a pharmaceutically acceptable salt thereof which process comprises
i)
a) obtaining impure clavulanic acid in a broth resulting from fermentation of a clavulanic acid - producing microorganism
b) contacting impure clavulanic acid or an alkali metal salt thereof in an organic solvent or solvents, with tertiary octylamine;
ii) isolating the tertiary-octylamine salt of clavulanic acid formed;
iii) converting the thus formed salt into clavulanic acid or a pharmaceutically acceptable salt or ester thereof characterised in that the organic solvent or solvents contain between 0.25 to 0.6g per litre of water.

16. A process according to any preceeding claim in which the tertiary-octylamine salt of clavulanic acid is recrystallised.

17. A process according to claim 22 in which the recrystallisation solvent is aqueous acetone.

18. A process according to any preceeding claim in which the tertiary-octylamine salt of clavulanic acid is converted into clavulanic acid or a pharmaceutically acceptable salt or ester thereof by ion-replacement which is performed by passing a solution of tertiary octylamine salt through a bed of a cation exchange resin in sodium, potassium or calcium form.

19. A process according to any one of claims 1 to 23 in which the tertiary-octylamine salt of clavulanic acid is converted into clavulanic acid or a pharmaceutically acceptable salt or ester thereof by ion-replacement which is performed by reaction of the protonated amine cation with potassium 2-ethylhexanoate or sodium 2-ethylhexanoate.
